# EUROPÄISCHE PATENTSCHRIFT

(11) **EP 1 503 705 B1**
(45) Veröffentlichungstag und Bekanntmachung des Hinweises auf die Patenterteilung: **23.01.2008**
(21) Anmeldenummer: 03752685.2
(22) Anmeldetag: 07.05.2003
(51) Int. Cl.: A61F 2/40

(54) **ENDOPROTHESE FÜR DEN ERSATZ EINES GELENKES, INSBESONDERE EINES SCHULTERGELENKES**
ENDOPROSTHESIS FOR REPLACING A JOINT, ESPECIALLY A SHOULDER JOINT
ENDOPROTHESE D'UNE ARTICULATION PROTHETIQUE, NOTAMMENT D'UNE ARTICULATION PROTHETIQUE D'EPAULE

(30) Priorität: 15.05.2002 CH 825022002
(43) Veröffentlichungstag der Anmeldung: 09.02.2005
(73) Patentinhaber: Horber, Willi, 8005 Zürich (CH)
(72) Erfinder: Horber, Willi, 8005 Zürich (CH)
(74) Vertreter: Walder, Martin Bernhard
(86) Internationale Anmeldenummer: PCT/CH2003/000295
(87) Internationale Veröffentlichungsnummer: WO 2003/096939

(56) Entgegenhaltungen:
- EP-A- 0 669 117
- EP-A- 0 712 617
- WO-A-01/22905
- WO-A-02/39931
- WO-A-02/39933
- FR-A- 2 773 469

## Beschreibung

### Technisches Gebiet der Erfindung

Die Erfindung betrifft eine Endoprothese für den Ersatz eines Gelenkes, insbesondere eines Schultergelenkes, gemäss dem Oberbegriff des Anspruchs 1. Eine solche Endoprothese weist ein Grundteil auf, in welchem eine sich zu ihrem Grund hin verjüngende Anlenkmulde mit einer rotationssymmetrischen Mantelfläche ausgebildet ist. Die Rotationsachse dieser Mantelfläche ist in einer Gelenkhalsrichtung gerichtet. In der Anlenkmulde ist ein Anlenkkörper angeordnet, der über eine zu einer Schwenkachse rotationssymmetrische Anlenkfläche an Berührungsstellen mit der Mantelfläche zusammenwirkt. Die Schwenkachse dieses Anlenkkörpers schneidet die Rotationsachse. Somit ist der Anlenkkörper um die Rotationsachse der Mantelfläche drehbar und um die Schwenkachse verschwenkbar am Grundteil angelenkt. Mit dem Anlenkkörper ist daher ein von diesem getragenes erstes Artikulationsteil in seiner Inklinations- und Rotationsstellung gegenüber dem Grundteil ausrichtbar und mit einem Befestigungsmittel in der gewählten Ausrichtung in der Anlenkmulde fixierbar. Das erste künstliche Artikulationsteil ist vorgesehen zur Artikulation mit einem natürlichen oder künstlichen zweiten Artikulationsteil.

### Stand der Technik

Aus der DE-299 18 589 U1 ist eine Endoprothese für ein Schultergelenk bekannt, bei welcher in einem Grundteil eine Anlenkmulde ausgebildet ist. Die Anlenkmulde ist als Konus ausgebildet. In dieser Anlenkmulde ist ein Drehstück angelenkt. Das Drehstück ist um die Achse des Konus drehbar und in jeglicher Drehstellung über eine Konusverklemmung mit dem Grundteil verbindbar. Am Drehstück ist ein Richtstück angelenkt. Das Richtstück trägt eine Kopfkalotte und ist um eine die Halsachse senkrecht schneidende Zylinderachse gegenüber dem Drehstück verschwenkbar. Diese Endoprothese erlaubt es, das Richtstück nicht über Kugeloberflächen, sondern über Oberflächen von axialen Rotationskörpem am Grundteil anzulenken. Dabei ist es möglich, die Rotationsstellung und die Inklinationsstellung der Kopfkalotte unabhängig voneinander einzustellen. Die Endoprothese weist ferner ein Befestigungsmittel zum Befestigen des Anlenkkörpers in der Anlenkmulde auf.

In der nachveröffentlichten internationalen Patentanmeldung WO02/39933 (PCT/CH01/00676) ist eine Endoprothese für ein Schultergelenk beschrieben, bei welchem im Kopfabschnitt des Grundteils ein rotationssymmetrischer Anlenkraum ausgebildet ist. In diesem Anlenkraum ist ein axialer Anlenkkörper angelenkt. Die Anlenkkörperachse steht dabei senkrecht zur Rotationsachse des Anlenkraumes. Der Anlenkraum besitzt in einem Beispiel einen kegelförmigen Boden. Dieser Boden dient als Klemmfläche, die mit einer axialsymmetrischen Anlenkfläche am Anlenkkörper zusammenwirkt. Damit eine Verkrallung von Anlenkfläche und Klemmfläche erreicht werden kann, ist vorgeschlagen, diese Flächen durch eine Anzahl von Kanten zu bilden. Bei dieser Endoprothese ist mit einfach herstellbaren rotationssymmetrischen Oberflächen erreicht, dass sowohl die Inklinations- als auch die Rotationsstellung einer mit dem Anlenkkörper verbundenen Kopfkalotte einstellbar ist. Dabei kommt der Gegenstand der WO02/39933 im Vergleich mit dem Gegenstand der DE-29918 589 U1 ohne Drehstück aus. Die Ausführungsbeispiele der WO02/39933 zeigen ein Spektrum möglicher Ausführungsvarianten, welches zumindest teilweise auch auf die vorliegende Erfindung übertragbar ist.

Bei den in der WO02/39933 beschriebenen Ausführungsbeispielen weist der Anlenkkörper eine lineare Anordnung der Berührungspunkte zwischen Anlenkkörper und Grundteil auf. Daher neigt der Anlenkkörper bei lockerem Befestigungsmittel dazu, auf eine entsprechende Krafteinwirkung hin mit der Anlenkfläche über die Klemmfläche abzurollen.

### Aufgabe der Erfindung

Es ist Aufgabe der Erfindung eine Endoprothese für den Ersatz eines Gelenkes vorzuschlagen, insbesondere eines Schultergelenkes, bei welchem ein axialer Anlenkkörper in einer rotationssymmetrischen Anlenkmulde in einer beliebigen Stellung bezüglich Inklination und Rotation feststellbar ist und bei welchem eine unbeabsichtigte Abrollbewegung zwischen Anlenkkörper und Anlenkmulde verhindert ist. In einer vorteilhaften Ausführungsform soll sich eine Klemmwirkung zwischen dem Anlenkkörper und der Anlenkmulde einstellen.

### Beschreibung der Erfindung

Diese Aufgabe ist erfindungsgemäss dadurch gelöst, dass bei einer eingangs erwähnten Endoprothese die im Kennzeichen des Patentanspruchs 1 angeführten Merkmale verwirklicht sind.

Die Anlenkfläche wirkt demnach an wenigsten drei voneinander getrennten Berührungsstellen mit der Mantelfläche zusammen. Die Anlenkfläche weist wenigstens eine um die Schwenkachse rotationssymmetrische Kreiskante auf, welche an zwei voneinander beabstandeten Berührungsstellen mit der Mantelfläche zusammenwirkt. Die beiden Berührungsstellen der Kreiskante sind bezüglich einer die Schwenkachse und die Rotationsachse enthaltenden Ebene derart angeordnet, dass eine Berührungsstelle auf der einen Seite und die andere Berührungsstelle auf der anderen Seite dieser Ebene liegt. Eine geometrisch lineare oder punktuelle Berührung ist in der Praxis unmöglich. Physikalisch werden immer flächige Berührungsstellen vorliegen. Daher werden lineare Kanten ebenfalls als Anlenkflächen betrachtet.

Der Radius der Kreiskante ist grösser als der Radius eines Scheitelkreises einer Schnittkurve zwischen einer senkrecht zur Schwenkachse liegenden Ebene durch die Berührungsstellen und einer Kegelmantelfläche. Die Schnittkurve ist eine Hyperbel. Die Kegelmantelfläche ist gebildet durch Rotation einer Geraden um die Rotationsachse, welche Gerade die Rotationsachse schneidet und eine Berührungsstelle berührt. Diese Gerade gehört zu einer durch eine erste und eine zweite Tangente an die Berührungsstelle definierten Tangentialebene. Die erste Tangente liegt in der senkrecht zur Schwenkachse liegenden Ebene und tangiert an der Berührungsstelle die Kreiskante. Die zweite Tangente liegt in einer senkrecht zur Rotationsachse liegenden Ebene und tangiert an der Berührungsstelle die Mantelfläche.

Vorteilhaft weist die Anlenkfläche wenigstens zwei um die Schwenkachse rotationssymmetrische Kreiskanten auf, von denen wenigstens eine an zwei voneinander beabstandeten Berührungsstellen mit der Mantelfläche zusammenwirkt. Dabei liegt die eine dieser Berührungsstellen auf der einen Seite einer die Schwenkachse und die Rotationsachse enthaltenden Ebene, während die andere Berührungsstelle auf der anderen Seite dieser Ebene liegt.

Die Mantelfläche ist vorteilhaft konisch ausgebildet. Sie weist demnach eine Kegelform mit geraden Mantellinien, eine Trompetenform mit zur Rotationsachse hin konvexen Mantellinien oder eine Eierbecherform mit zur Rotationsachse hin konkaven Mantellinien auf. Im Fall einer Kegelform weist die Kreiskante an den Berührungsstellen zwischen der Kreiskante und der Mantelfläche einen Radius bezüglich der Schwenkachse auf, der grösser ist als der Scheitelradius einer Schnittkurve zwischen der Mantelfläche und einer senkrecht zur Schwenkachse verlaufenden Ebene durch die Berührungsstellen. In beiden Fällen mit gebogenen Mantellinien muss der Radius der Kreiskante grösser sein als der Scheitelradius einer Schnittkurve zwischen der senkrecht zur Schwenkachse verlaufenden Ebene durch die Berührungspunkte der Kreiskante und einer die Mantelfläche berührenden Kegelmantelfläche. Diese Kegelmantelfläche wird erhalten durch Rotation einer Tangente an die Mantellinie an der Berührungsstelle um die Rotationsachse. Dadurch wird erreicht, dass der Anlenkkörper nicht lediglich linear mit der Mantelfläche zusammenwirkt, sondern wenigstens eine Dreipunktabstützung aufweist. Diese Anordnung erlaubt auch durch entsprechende Wahl der Mantelfläche und der Anlenkfläche eine Klemmwirkung zwischen Anlenkmulde und Anlenkkörper zu erreichen.

Erfüllen zwei oder gar mehrere Kreiskanten obige Kriterien, so liegen von den Berührungsstellen je zwei oder mehr auf einer und zwei oder mehr auf der anderen Seite einer die Rotations- und die Schwenkachse enthaltenden Ebene. Somit ist eine viereckige Anordnung der Berührungsstellen erreicht. Diese nicht lineare Anordnung der Berührungspunkte verhindert, ebenso wie die Dreipunktabstützung, dass der Anlenkkörper auf der Mantelfläche abrollt. Die Radien der Kreiskanten können indes unterschiedlich sein. Bei zwei Kreiskanten mit jeweils gleichen Radien schneidet die Schwenkachse die Rotationsachse für gewöhnlich senkrecht

Die beiden zueinander beabstandeten Kreiskanten müssen indes nicht in Parallelebenen zur Rotationsachse liegen. Der Anlenkkörper kann in einem beschränkten Mass auch quer zur Schwenkachse verschwenkt werden. Dabei ergibt sich eine gewisse seitliche Verschiebung (Offset) des Anlenkkörpers gegenüber der Rotationsachse. Dieser Offset kann bei der Ausrichtung des Anlenkkörpers genutzt werden, um beispielsweise die Lage der Gelenkhalsachse bzw. des auf den Anlenkkörper aufgesetzten Artikulationsteils in einem Schultergelenk zu optimieren.

Die Mantelfläche der Anlenkmulde kann trompetenförmig, eierbecherförmig oder kegelförmig gestaltet sein. Die Mantelfläche kann vollflächig, aber auch durch eine oder mehrere Ringkanten gebildet sein. Die Ringkante kann geschlossen oder auch eine Gewindekante sein. Wenn die Mantelfläche wenigstens eine Ringkante aufweist, besitzt die Kreiskante an den Berührungsstellen zwischen Kreiskante und Ringkante vorteilhaft einen Radius bezüglich der Schwenkachse, der grösser ist als der Scheitelradius einer Schnittkurve zwischen der senkrecht zur Schwenkachse liegenden Ebene durch die Berührungsstellen einer Kreiskante und einer Kegelmantelfläche. Zur Konstruktion dieser Kegelmantelfläche wird eine die Tangente an die Ringkante und die Tangente an die Kreiskante an der Berührungsstelle enthaltende Ebene mit der Rotationsachse geschnitten. Eine Gerade durch den so erhaltenen Schnittpunkt auf der Rotationsachse und die Berührungsstelle wird um die Rotationsachse rotiert. Die daraus resultierende Kegelmantelfläche wird nun mit einer senkrecht zur Schwenkachse liegenden Ebene durch die Berührungsstellen geschnitten. Wenn der Scheitelradius dieser Schnittkurve kleiner ist als der Radius der Kreiskante, so liegt die Kreiskante an zwei Punkten an der Ringkante an.

Bei einer Ringkante geschieht bei einer Verschwenkung des Anlenkkörpers quer zur Schwenkachse kein Offset. Dies kann genutzt werden, indem eine konische Mantelfläche mit einer Ringkante kombiniert wird, falls die Möglichkeit des Offset ausgeschlossen werden soll. Bei so kombinierter Mantelfläche liegt der Anlenkkörper lediglich an allen möglichen Berührungspunkten an, wenn die Schwenkachse in einem vorbestimmten Winkel, gewöhnlich senkrecht zur Rotationsachse steht. Durch Festziehen des Befestigungsmittels wird daher der Anlenkkörper in diese Lage gebracht

Vorteilhaft liegt der Winkel zwischen der wirklichen Mantelfläche oder der konstruierten Kegelmantelfläche und der Rotationsachse an den Berührungsstellen in einem Bereich von 0 bis 30°, vorzugsweise zwischen 2 und 20°, besonders bevorzugt zwischen 5 und 15°. Dadurch wird eine Klemmwirkung zwischen Anlenkfläche und Mantelfläche erreichbar. Die Klemmwirkung ist bei Winkeln zwischen 0 und 5 Grad am höchsten, während die Einsinktiefe in diesen Winkelbereichen grössere Toleranzen aufweist. Bei Winkeln ab 15 Grad ist die Klemmwirkung reduziert, jedoch ist die Einsinktiefe präziser im Voraus bestimmbar. Es sind jedoch auch grössere Winkel unter 90° möglich, falls auf die Klemmwirkung verzichtet wird. Die Klemmwirkung erlaubt es, den Anlenkkörper durch leichtes Eindrücken in die Anlenkmulde provisorisch zu fixieren. Dadurch kann die Stellung der Halsachse kontrolliert werden, bevor diese Stellung durch Festziehen des Befestigungsmittels fixiert und dabei die Anlenkfläche oder die Mantelfläche verformt wird.

Dank den beim Festziehen des Befestigungsmittels relativ hohen Druckkräften an den Berührungsstellen wird wenigstens eine von Anlenkfläche und Mantelfläche verformt. Je nach Materialpaarung kann erreicht werden, dass sich die Anlenkfläche an den Berührungsstellen in die Mantelfläche eingräbt oder dass die Anlenkfläche, das heisst die Kreiskanten am Anlenkkörper, verformt werden. In letzterem Fall plättet die Mantelfläche die Kreiskanten an den Berührungsstellen ab. Dank diesen Verformungen an den Berührungsstellen wird eine Verschwenkung und eine Rotation des Anlenkkörpers um die Schwenkachse bzw. um die Rotationsachse zuverlässig verhindert. Die Härte des für den Anlenkkörper, das Befestigungsmittel bzw. die Anlenkmulde verwendeten Materials kann deshalb entsprechend der beabsichtigten Verformung gewählt werden. Wenn auch diese Teile aus dem gleichen oder aus gleich hartem Material hergestellt sein können, ist eine ungleiche Paarung von Vorteil. Besonders vorteilhaft ist der Anlenkkörper aus weicherem Material als die Anlenkmulde und das Befestigungsmittel hergestellt

Eine weitere Möglichkeit zur Erhöhung der Verklemmfestigkeit ist die Strukturierung der Kreiskanten und/oder der Mantelfläche durch vorstehende scharfe Kanten oder Spitzen, durch Kerben, Rillen oder Rasterreliefs. Dabei ist es vorteilhaft, die Strukturierungen der beiden Flächen aufeinander abzustimmen, so dass sie sich beim Festklemmen gegenseitig verzahnen.

Vorteilhaft weist der Anlenkkörper eine zentrale Bohrung mit einem rotationssymmetrischen Grund auf. Das Rotationszentrum oder die Rotationsachse des Grundes fällt vorteilhaft mit der Schwenkachse zusammen. Im Grund der Bohrung im Anlenkkörper ist eine Öffnung ausgebildet, durch welche hindurch ein Befestigungsmittel verläuft. Die Öffnung ist insbesondere ein Langloch mit Längserstreckung senkrecht zur Schwenkachse. Das Befestigungsmittel ist vorteilhaft eine Schraube zum Verbinden von Anlenkkörper und Grundteil. Es sind jedoch auch andere Befestigungsmittel möglich, wie zum Beispiel Spreizbolzen oder Nieten. Der Grund kann zylindrisch, doppelkegelförmig oder spindelförmig ausgebildet sein. Aus herstellungstechnischen und mechanischen Gründen wird ein sphärischer Grund bevorzugt. Diese rotationssymmetrische Ausbildung des Grundes erlaubt, einen Schraubenkopf mit einer kreisförmigen Kante gegen den Grund festzuziehen. Die kreisförmige Kante gräbt sich dabei in den Grund ein. Ist der Grund zylindrisch mit einer Zylinderachse, welche mit der Schwenkachse des Anlenkkörpers zusammenfällt, so gräbt sich der Schraubenkopf an zwei Stellen ein. Diese Stellen liegen in einer Ebene senkrecht zur Schwenkachse und in einem Abstand zur Schwenkachse. Dadurch wird einer Schwenkbewegung um die Schwenkachse des Anlenkkörpers zusätzlich entgegengewirkt.

Zur zusätzlichen Sicherung der Klemmung können geeignete Hilfsmittel wie z.B. Anpressdeckel, Konterschrauben oder -muttern, plastisch oder elastisch verformbare Einlagen zwischen Schraubenkopf und Grund oder in den Gewindegängen der Schraubverbindung eingesetzt werden.

Die beschriebene Geometrie erlaubt innerhalb gewisser Grenzen auch eine Verschwenkung der Schwenkachse um eine dritte Achse senkrecht zur Schwenkachse und zur Rotationsachse. Eine Begrenzung für diese Verschwenkung bildet der Winkel der Mantelfläche an den Berührungsstellen. Sobald die Schnittkurve zwischen der Normalebene zur Schwenkachse durch die Berührungsstellen und der oben definierten Kegelmantelfläche eine Parabel ergibt, ist eine sichere Verklemmung zwischen Anlenkkörper und Anlenkmulde nicht mehr herbeiführbar. Solange jedoch die Schnittkurve eine Hyperbel ist, kann der Anlenkkörper sicher in der Anlenkmulde befestigt werden. Dabei stellt sich, wie oben beschrieben, unter Umständen ein Offset ein. Soll dieser Offset genutzt werden können, muss die Öffnung einen Öffnungsdurchmesser aufweisen, der grösser ist als der Durchmesser der Befestigungsschraube.

Anstelle oder zusätzlich zu einer Befestigung durch eine Bohrung des Anlenkkörpers können auch andere Befestigungsmittel eingesetzt werden, wie zum Beispiel Schraubdeckel, Anpressplatten, Überwurfmuttern und dergleichen, wie sie u.a. in den Beispielen der WO02/39933 beschrieben sind. Die Befestigung am Grund einer Bohrung im Anlenkkörper weist den Vorteil auf, dass der Anlenkkörper sehr kompakt ausgebildet sein kann. Dies ermöglicht eine geringe Querschnittfläche des Anlenkkörpers und so auch einen geringen Durchmesser der Anlenkmulde. Daraus ergibt sich wieder ein geringer Aussendurchmesser des Kopfabschnittes, in welchem die Anlenkmulde ausgebildet ist. Zur weiteren Reduktion des Aussendurchmessers weist vorteilhaft der Öffnungsrand der Anlenkmulde eine den Öffnungsrand umlaufende Verstärkungsrippe auf. Dadurch kann die Wandstärke des Kopfabschnitts minimal dimensioniert sein, ohne dass die Gefahr besteht, dass die Wandung unter Belastung nachgibt oder gar reisst. Vorteilhafte Ausführungsformen der Erfindung sind in den abhängigen Ansprüchen beschrieben.

### Kurzbeschreibung der Figuren

In den Figuren sind Ausführungsbeispiele für Schulterprothesen dargestellt. Es zeigt:
- Fig. 1: eine Ansicht von Kopfabschnitt, Anlenkkörper und Befestigungsschraube gemäss einem ersten Ausführungsbeispiel,
- Fig. 2: ein Seitenansicht des Anlenkkörpers von Figur 1,
- Fig. 3: eine Aufsicht auf den Anlenkkörper von Fig. 1 und 2,
- Fig. 4: eine Aufsicht auf den Kopfabschnitt mit Anlenkmulde,
- Fig. 5: eine Aufsicht auf den Kopfabschnitt mit eingesetztem Anlenkkörper,
- Fig. 6: eine Ansicht der zusammengestellten Endoprothese ohne Kopfkalotte und ohne Gelenkpfanne,
- Fig. 7: einen Vertikalschnitt durch die Endoprothese gemäss Figur 6,
- Fig. 8: einen Schnitt gemäss Figur 7, jedoch mit Kopfkalotte und Glenoid,
- Fig. 9: einen schematischen Schnitt senkrecht zur Schwenkachse der Anlenkfläche und durch zwei Berührungsstellen der Anlenkfläche mit der Mantelfläche der Anlenkmulde,
- Fig. 10: einen Schnitt durch eine zweistufige Anlenkmulde 22 gemäss einem zweiten Ausführungsbeispiel mit darin angelenktem Anlenkkörper mit vier Kreiskanten,
- Fig. 11: eine schematische Darstellung der Anordnung der Berührungsstellen zwischen Anlenkmulde und Anlenkkörper,
- Fig. 12: eine Ansicht von unten des Anlenkkörpers gemäss Figur 10,
- Fig. 13: eine Draufsicht auf den Anlenkkörper gemäss Fig. 10 und 12.
- Fig. 14: eine geometrische Darstellung der Geometrie, welche zwei Berührungspunkte zwischen einer Kreiskante der Anlenkfläche und der Mantelfläche gewährleistet,
- Fig. 15: ein Ausführungsbeispiel mit einer Dreipunktauflage zwischen Anlenkkörper 15 und Mantelfläche 25,
- Fig. 16: das Ausführungsbeispiel gemäss Figur 15, jedoch mit um 180° gedreht eingesetztem Anlenkkörper 15.

### Beschreibung der Ausführungsbeispiele

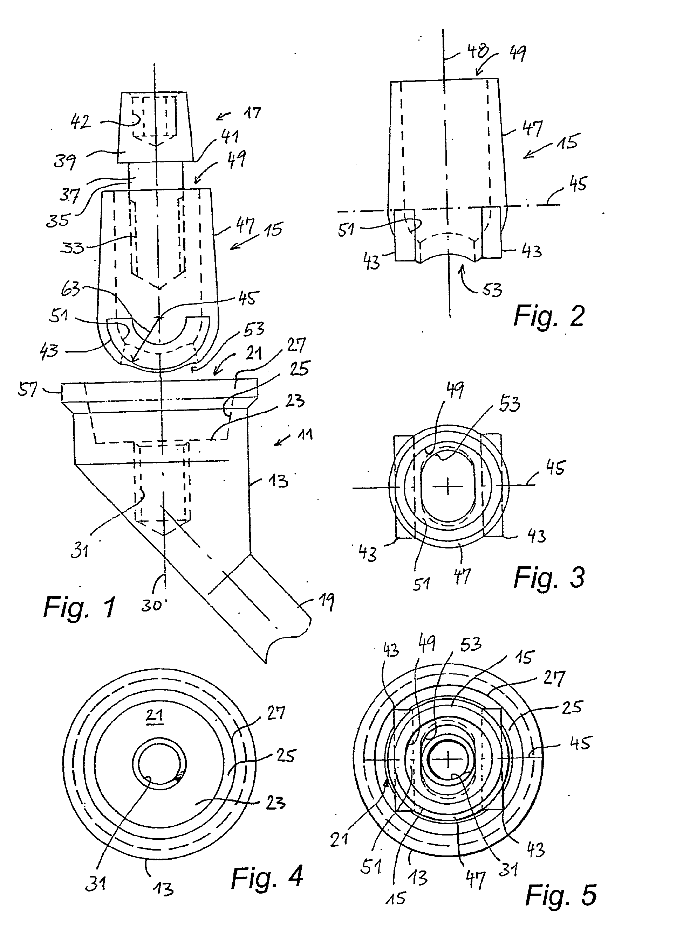

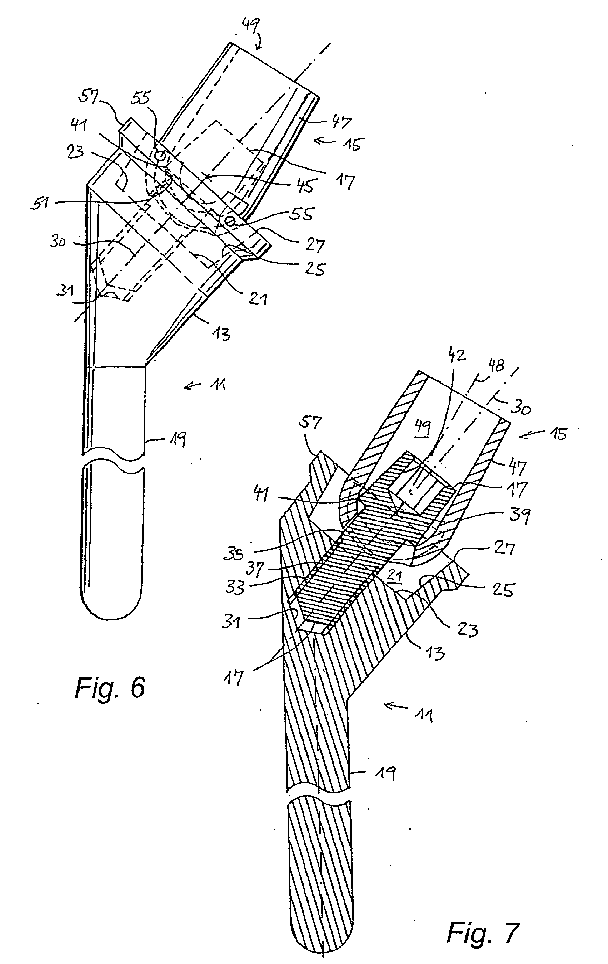

In Figur 1 sind Grundteil 11, Anlenkkörper 15 und Befestigungsschraube 17 voneinander gelöst dargestellt. Die selben Teile sind in Figur 6 und 7 zusammengesetzt dargestellt. Das Grundteil 11 zum Einsetzen in die Diaphyse, bzw. Metaphyse des Humerus besitzt einen Stielabschnitt 19, und einen Kopfabschnitt 13. Im Kopfabschnitt 13 des Grundteils ist eine Anlenkmulde 21 ausgebildet. Die Anlenkmulde 21 ist als Kegelstumpf-Becher ausgebildet. Sie besitzt einen ebenen Muldenboden 23 und eine kegelförmige Mantelfläche 25 als Muldenwandung. An ihrem Öffnungsrand 27 weist die Anlenkmulde 21 einen grösseren Durchmesser auf als am Muldenboden 23. Die Winkel zwischen kegelförmiger Mantelfläche 25 und Rotationsachse 30 beträgt 10 Grad. Bevorzugte Winkel liegen zwischen 5 und 15 Grad, doch sind auch grössere oder kleinere Winkel möglich. Im Muldenboden 23 ist auf der Rotationsachse 30 eine Gewindebohrung 31 vorhanden. Die Dimension und die Gewindeneigung der Gewindebohrung 31 ist auf die Befestigungsschraube 17 abgestimmt. Diese besitzt einen Schaft 37 mit einem unteren Gewindeabschnitt 33 und einem oberen Schaftbereich 35 ohne Gewinde. Weiter weist die Befestigungsschraube 17 einen Schraubenkopf 39 auf. Der Schraubenkopf 39 besitzt eine Kopfkante 41 mit einem grösseren Radius als dem des Schafts 37. Am Schraubenkopf 39 ist eine Angriffsfläche 42 angeordnet, welche auf ein Werkzeug zum Anziehen der Befestigungsschraube 17 abgestimmt ist. Zwischen Schraubenkopf 39 und Anlenkmulde 21, insbesondere zwischen der Kopfkante 41 und der kegelförmigen Mantelfläche 25, kann der Anlenkkörper 15 festgeklemmt werden.

Der Anlenkkörper 15 passt in die Anlenkmulde 21. Der Anlenkkörper 15 besitzt Anlenkflächen 43, die rotationssymmetrisch zur Schwenkachse 45 ausgebildet sind. Im vorliegenden Beispiel sind die Anlenkflächen zwei Kreiskanten 43. Dieses Beispiel ist jedoch nicht limitierend. Die Anlenkflächen 43 können durchgehend oder unterbrochen ausgebildet sein. Unterbrüche in der Anlenkfläche werden durch Einschnitte oder Kerben in der Fläche oder Kante erreicht. Die Anlenkfläche kann demnach auch aus einer Reihe von Spitzen bestehen. Die Anlenkmulde 21 kann in Abkehr von der reinen Kegelform trompeten- oder eierbecherförmig gestaltet sein. In diesem Falle sind die Mantellinien nicht wie beim Kegel geradlinig, sondern konvex, bzw. konkav gebogen. Die Mantelfläche kann überdies z.B. Kreisrillen, Gewinderillen oder Mantellinienrillen aufweisen. Mantellinienrillen sind vorteilhaft derart ausgebildet, dass die Wandungen der Rillen an der Stirnseite und der Mantelseite des Anlenkkörpers 15 anliegen. Dies begünstigt eine Verklemmung des Anlenkkörpers in der Anlenkmulde 21. Vorteilhaft ist dabei der Material der Mantelfläche weicher als das Material des Anlenkkörpers.

Die Mantelfläche kann mit einem dreidimensionalen Rasterrelief geprägt sein. Die Mantelfläche kann durch drei oder mehrere zylindrische und konzentrische Bohrungen mit fein abgestuften, sukzessive abnehmenden Durchmessern gebildet sein. Dabei schneiden beim Einpressen des Anlenkkörpers 15 in die Bohrungen deren Öffnungskanten in die Anlenkflächen 43 des Anlenkkörpers 15. Die Anlenkmulde 21 kann zwei oder mehrere Kegelmantelflächen 25 in einer abgestuften Reihe aufweisen. Diese können mit den selben oder mit jeweils verschiedenen Anlenkflächen 43 des Anlenkkörpers 15 zusammenwirken.

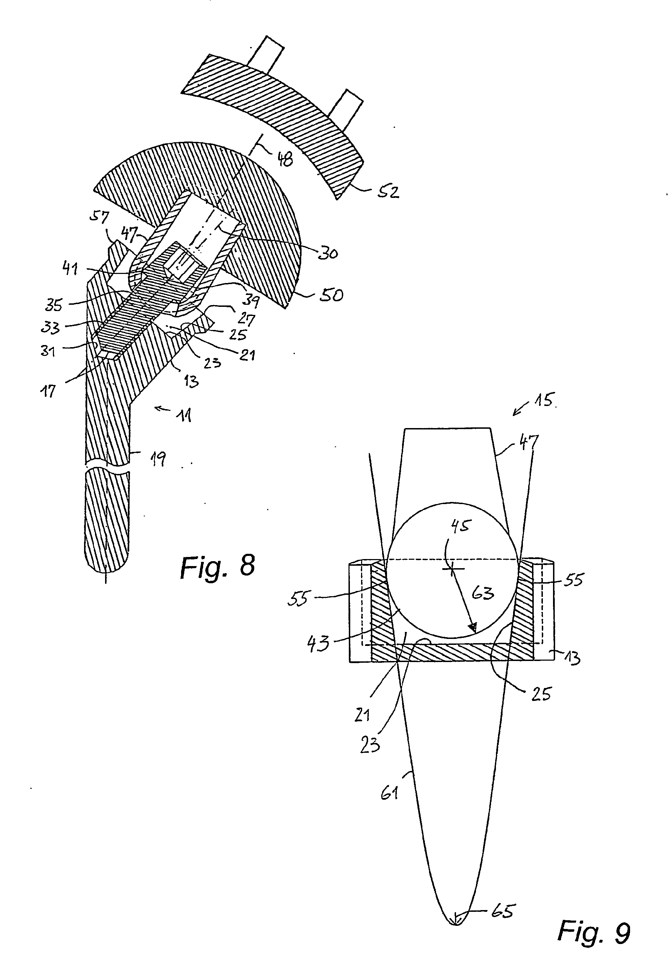

Der Anlenkkörper 15 weist eine äussere Konusfläche 47 konzentrisch um eine Halsachse 48 auf (Fig. 2), welche Konusfläche 47 zusammen mit der Halsachse 48 von der Richtung der Rotationsachse 30 abweichend gerichtet werden kann. Auf diese Konusfläche 47 kann eine Kopfkalotte 50 (Fig. 8) aufgesetzt werden, die mit einem natürlichen oder künstlichen Glenoid 52 (Fig. 8) zusammenwirkt. Konzentrisch zur Konusfläche 47 ist eine Bohrung 49 im Anlenkkörper 15 vorhanden. Diese Bohrung 49 ist im Wesentlichen eine Sackbohrung mit einem sphärischen Grund 51. Der Grund 51 kann auch zylindrisch oder einer Hohlkugeloberfläche lediglich angenähert sein. Im Grund 51 ist eine Langlochbohrung 53 vorhanden. Diese weist eine Breite auf, die zumindest dem Durchmesser des Schaftes 37 der Befestigungsschraube 17 entspricht. Die Länge des Langloches 53 erstreckt sich in Schwenkrichtung um die Schwenkachse 45 und bestimmt das Ausmass der Verschwenkbarkeit des Anlenklörpers. Die Verschwenkbarkeit bezüglich der Rotationssachse 30 beträgt im vorliegenden Beispiel in beide Richtungen etwa 10 Grad, wie aus den Fig.6 und 7 ersichtlich ist.

In Figuren 4 und 5 ist eine Aufsicht auf die Anlenkmulde 21 dargestellt. In Figur 4 ist die Anlenkmulde leer. In Figur 5 ist der Anlenkkörper 15 in die Anlenkmulde 21 eingesteckt. Aus Figur 5 ist ersichtlich, dass die Anlenkflächen 43 die kegelförmige Mantelfläche 25 an vier Stellen berühren. Diese Stellen liegen praktisch auf den Eckpunkten eines Rechteckes mit in diesem Beispiel beinahe gleichen Seitenlängen. In Figur 6 sind die Berührungsstellen 55 mit Kreisen hervorgehoben. Die Schnittkurve der senkrecht zur Schwenkachse liegenden und durch die Berührungsstellen gehenden Ebene mit der kegelförmigen Mantelfläche 25 ist eine Hyperbel 61 (Fig. 9). Der Scheitelradius 65 dieser Hyperbel 61 ist kleiner als der Radius 63 der Anlenkfläche 43. Die Berührungsstellen 55 liegen mit zunehmender Einstecktiefe weiter vom Öffnungsrand 27 der Anlenkmulde 21 entfernt. Mit einer Zunahme der Einstecktiefe rücken die Berührungsstellen 55 näher an die Rotationsachse 30.

Die für eine dauerhafte Verkrallung und Verklemmung von Anlenkkörper und Anlenkmulde benötigte Druckkraft wird durch die Schraubenverbindung erreicht. Zwischen der Kopfkante 41 und der kegelförmigen Mantelfläche 25 ist der Anlenkkörper 15 eingeklemmt. Damit die an den vier Berührungsstellen 55 auftretenden Kräfte nicht zu einer Deformation des Kopfabschnittes führen oder Risse in der Wandung der Anlenkmulde 21 verursachen, ist der Öffnungsrand 27 der Anlenkmulde 21 mit einer ringförmig umlaufenden Verstärkungsrippe 57 verstärkt.

Die Figur 8 ist gegenüber der Figur 7 lediglich um die beiden Artikulationsteile 50 und 52, in diesem Beispiel um eine Kopfkalotte 50 und das künstliche Glenoid 52 ergänzt. Figur 9, welche einen schematischen Schnitt parallel zur Rotationsachse 30 durch Anlenkstellen 55 darstellt, zeigt eine Hyperbel 61. Diese Hyperbel 61 ist die Schnittkurve zwischen einer senkrecht zur Schwenkachse 45 liegenden Ebene durch die zwei Berührungsstellen 55 der Kreiskante 43 mit dem Radius 63 und der zu einem Kegel ergänzten, kegelstumpfförmigen Mantelfläche 25. Bei Mantelflächen, die von der Kegelform abweichen, ergibt die Schnittkurve eine andere geometrische Kurve.

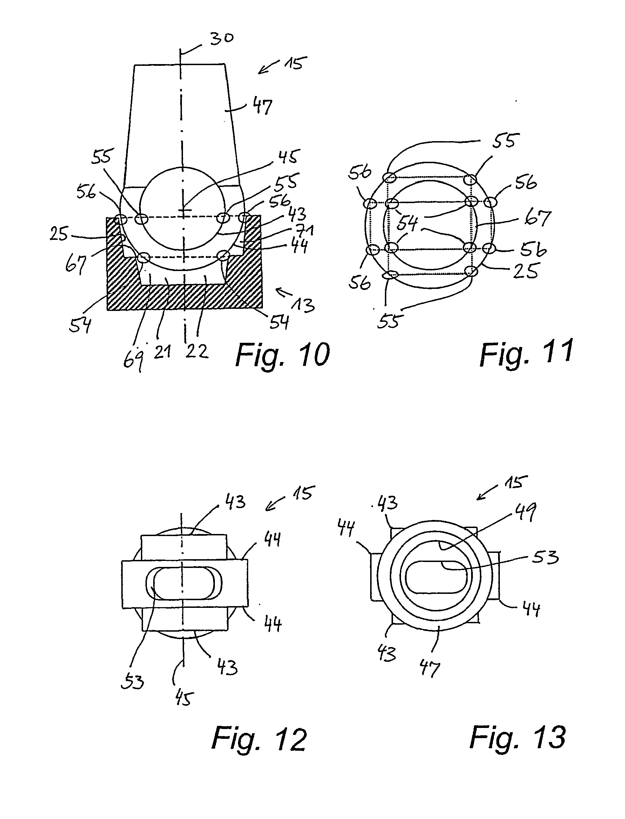

Das in den Figuren 10 bis 13 gezeigte zweite Ausführungsbeispiel zeigt eine abgestufte Anlenkmulde 22 und einen Anlenkkörper 15 mit zweimal zwei Kreiskanten 43 und 44. Auf Grund der Abstufung der Anlenkmulde 22 liegt in der Anlenkmulde eine Ringkante 67 am Öffnungsrand der inneren, kleineren Bohrung 69 vor. Die Kreiskanten 43 wirken lediglich mit der Mantelfläche 25 der grösseren Bohrung 71 zusammen. Mit vier Berührungsstellen 55 (mit kleinen Ellipsen hervorgehoben) zwischen Kreiskante 43 und Mantelfläche 25 wird wie beim ersten Ausführungsbeispiel beim Eindrücken des Anlenkkörpers 15 in die Anlenkmulde 22 eine Klemmwirkung zwischen diesen Teilen erreicht.

Die Kreiskanten 44 jedoch weisen einen grösseren Radius auf als die Kreiskanten 43 und weisen einen kleineren Abstand zueinander auf als diese. Die Kreiskanten 44 wirken sowohl mit der Mantelfläche 25 als auch mit der Ringkante 67 zusammen. An den Berührungsstellen 56 zwischen der Kreiskante 44 und der Mantelfläche 25 ist der Winkel zwischen der Mantelfläche und der Rotationsachse 30 gering.

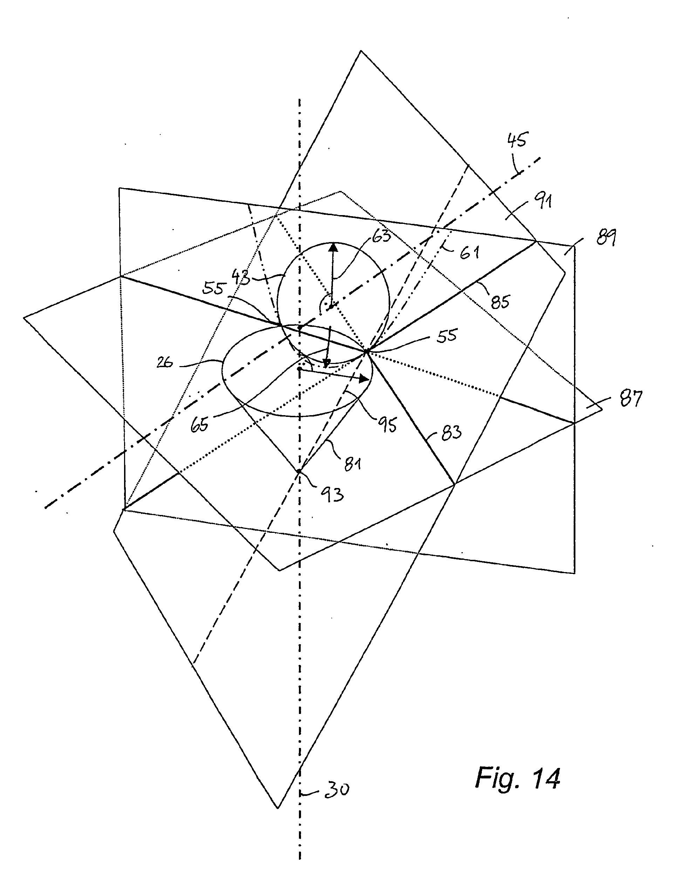

An den Berührungsstellen 54 zwischen der Ringkante 67 und der Kreiskante 44 weist eine Schnittkurve zwischen der Rotationsfläche, gebildet durch die Rotation der Kegelmantellinie 95 gemäss Figur 14 um die Rotationsachse 30, und einer die Kreiskante 44 enthaltenden Ebene einen Scheitelradius auf, der kleiner ist als der Radius der Kreiskante 44. Der Winkel zwischen dieser Rotationsfläche und der Rotationsachse ist an den Berührungsstellen 54 wesentlich grösser als der Winkel der Mantelfläche 25 zur Rotationsachse30. Somit wird mit den acht Berührungsstellen 55, 56 an der Mantelfläche 25 eine Klemmwirkung erreicht, während die vier Berührungsstellen 54 an der Ringkante 67 die Eindringtiefe begrenzen.
In der schematischen Darstellung des ersten und des zweiten Ausführungsbeispiels sind die Kreiskanten 43,44 als Kanten von zylindrischen Körpern dargestellt. Diese Kreiskanten können jedoch auch auf anders geformten Körpern ausgebildet sein.

In der Figur 11 sind zwei Kreise dargestellt. Diese Kreise stellen die Schruttkreise in den Ebenen der jeweiligen Berührungsstellen 54,55,56 dar. Die Berührungsstellen bilden die Eckpunkte von drei Rechtecken. Ein Rechteck ist gebildet durch die Berührungsstellen 54 zwischen der Ringkante 67 und den Kreiskanten 44. Ein zweites Rechteck ist gebildet durch die Berührungsstellen 55 zwischen der Mantelfläche 25 und den Kreiskanten 43. Ein drittes Rechteck ist gebildet durch die Berührungsstellen 56 zwischen der Mantelfläche 25 und den Kreiskanten 44. Durch diese zwölf Berührungsstellen ist eine stabile Abstützung des Anlenkkörpers 15 in der Anlenkmulde 22 gewährleistet.

Wie im ersten Beispiel (Fig.1 bis 8) ist auch im zweiten Ausführungsbeispiel (Fig.10 bis 13) am Anlenkkörper 15 ein Konus 47 zur Aufnahme eines Artikulationsteils ausgebildet. Dieser Konus kann um die Schwenkachse 45 verschwenkt und um die Rotationsachse 30 gedreht werden. Der Anlenkkörper 15 weist zum Feststellen des Anlenkkörpers 15 in der Anlenkmulde ebenfalls eine zentrische Bohrung 49 im Konus 47 auf, welche einen sphärischen Grund und darin eine Langlochbohrung 53 aufweist (vergl. Fig.12 und 13). Durch das Langloch 53 hindurch ist eine Schraube mit dem Kopfabschnitt 13 des Grundteils 11 verbindbar. Zur Sicherung der Schraubklemmung können geeignete Hilfsmittel wie Pressdeckel, Konterschrauben, verformbare Einlagen etc. vorgesehen sein. Als Befestigungsmittel kommt aber auch z.B. eine Überwurfmutter in Frage, die über ein Gewinde an der Aussenseite des Kopfabschnittes 13 an gezogen wird und dabei die Kreiskante 43 und damit den Anlenkkörper 15 in die Anlenkmulde 21 presst

In Figur 14 ist die im Anspruch 2 definierte Geometrie dargestellt Diese Geometrie gewährt, dass der Anlenkkörper mit einer Kreiskante 43 mit dem Radius 63 an der Mantelfläche 25 an zwei Berührungspunkten 55 anliegt. Die Berührungspunkte 55 einer Kreiskante 43 liegen auf einer Kreislinie 26 um die Rotationsachse 30. Die Kreislinie 26 ist die Schnittlinie einer Ebene 87 senkrecht zur Rotationsachse 30 durch die Mantelfläche 25 des Kegelmantels 81 und ist somit Teil der Mantelfläche 25. Unabhängig davon, welche Form die Mantelfläche 25 besitzt, ist diese Kreislinie 26 massgebend für die Verklemmung zwischen Anlenkkörper und Anlenkmulde. Vereinfachend gesagt ist der die Klemmwirkung bestimmende Konuswinkel der halbe Scheitelwinkel des dargestellten Kegelmantels 81. Dieser Kegelmantel 81 fällt im Fall einer kegelförmigen Mantelfläche 25 mit der Mantelfläche zusammen. In allen anderen Fällen jedoch nicht. Dieser Kegelmantel 81 ist folgendermassen konstruierbar: Es werden zwei Tangenten 83,85 an die Berührungsstelle 55 gelegt. Die eine Tangente 83 liegt in einer Normalebene 87 zur Rotationsachse 30, die andere Tangente 85 liegt in einer Normalebene 89 zur Schwenkachse 45. Die beiden Tangenten 83,85 definieren eine Tangentialebene 91 an die Berührungsstelle 55. Diese Tangentialebene 91 schneidet die Rotationsachse 30 im Kegelscheitelpunkt 93. Eine Rotation der Kegelmantellinie 95 um die Rotationsachse 30, welche Kegelmantellinie 95 durch den Kegelscheitelpunkt 93 und die Berührungsstelle 55 läuft, ergibt den Kegelmantel 81. Die Schnittkurve 61 zwischen dem Kegelmantel 81 und der Normalebene 89 zur Schwenkachse 45 ist eine Hyperbel mit einem Scheitelradius 65 kleiner als der Radius 63 der Kreiskante 43.

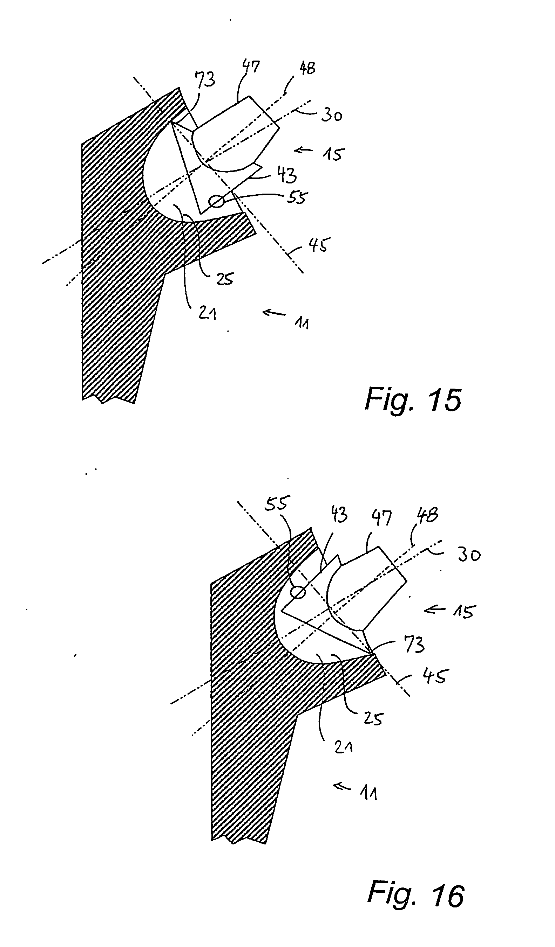

Diese Geometrie muss lediglich bei einer einzigen Kreiskante 43 erfüllt sein. Die Figuren 15 und 16 zeigen ein Ausführungsbeispiel mit einer einzigen Kreiskante 43 und einer Spitze 73. Die so erhaltene Dreipunktauflage erlaubt, wie auch eine Vierpunktauflage, eine Verschwenkung des Anlenkkörpers 15 um eine dritte Achse senkrecht zur Schwenkachse 45 und zur Rotationsachse 30. Bei einer Verschwenkung um diese dritte Achse tritt eine Verschiebung des Anlenkkörpers in Richtung der Schwenkachse 30 auf. Dieser Offset ist aus den beiden Darstellungen von Figur 15 und 16 ersichtlich. Beide Anlenkkörper 15 sind um denselben Winkel um die dritte Achse verschwenkt. Je nachdem ob die Spitze 73 dabei angehoben oder gesenkt wird, ergibt sich jedoch eine andere Lage der Gelenkhalsachse 48 bezüglich der Rotationsachse 30.

## Patentansprüche

1. Endoprothese für den Ersatz eines Gelenkes, insbesondere eines Schultergelenkes, mit
- einem in einem Knochen verankerbaren Grundteil (11),
- einer im Grundteil (11) angeordneten zum Muldengrund (23) hin verjüngend ausgebildeten Anlenkmulde (21) mit einer rotationssymmetrischen Mantelfläche (25),
- einem Anlenkkörper (15) in der Anlenkmulde (21), welcher Anlenkkörper (15) ein erstes Artikulationsteil (50) zur Artikulation mit einem zweiten natürlichen oder künstlichen Artikulationsteil (52) trägt und eine Anlenkfläche (43) aufweist, die mit der Mantelfläche (25) der Anlenkmulde (21) an Berührungsstellen (55) zusammenwirkt, welche Anlenkfläche (43) an jeder Berührungsstelle (55) rotationssymmetrisch bezüglich einer einzigen, die Rotationsachse (30) schneidenden Schwenkachse (45) ausgebildet ist,
- und mit einem Befestigungsmittel (17) zum Befestigen des Anlenkkörpers (15) in der Anlenkmulde (21),
**dadurch gekennzeichnet,**
- **dass** die Anlenkfläche (43) an wenigstens drei voneinander getrennten Berührungsstellen (55) mit der Mantelfläche (25) zusammenwirkt,
- **dass** die Anlenkfläche (43) wenigstens eine um die Schwenkachse (45) rotationssymmetrische Kreiskante (43) aufweist, welche an zwei der wenigstens drei voneinander beabstandeten Berührungsstellen (55) mit der Mantelfläche (25) zusammenwirkt,
- und **dass** die eine dieser Berührungsstellen (55) der Kreiskante (43) auf der einen Seite einer die Schwenkachse (45) und die Rotationsachse (30) enthaltenden Ebene und die andere Berührungsstelle (55) der Kreiskante (43) auf der anderen Seite dieser Ebene liegt.

2. Endoprothese nach Anspruch 1, **dadurch gekennzeichnet, dass** der Radius (63) der Kreiskante (43) grösser ist als der Radius (65) eines Scheitelkreises einer Schnittkurve (61) zwischen einer senkrecht zur Schwenkachse (45) liegenden Ebene (89) durch die Berührungsstellen (55) der Kreiskante (43) und einem Kegelmantel (81), gebildet durch Rotation um die Rotationsachse (30) einer die Rotationsachse (30) und eine Berührungsstelle (55) schneidenden Geraden (95), welche Gerade (95) zu einer durch eine erste Tangente (85) und eine zweite Tangente (83) an die Berührungsstelle (55) definierten Tangentialebene (91) gehört, welche erste Tangente (85) in der senkrecht zur Schwenkachse (45) liegenden Ebene (89) die Kreiskante (43) tangiert und welche zweite Tangente (83) in einer senkrecht zur Rotationsachse (30) liegenden Ebene (87) die Mantelfläche (25) tangiert.

3. Endoprothese nach Anspruch 1 oder 2, **dadurch gekennzeichnet, dass** die Mantelfläche (25) kegelförmig mit geradlinigen Mantellinien oder trompetenförmig mit zur Rotationsachse (30) hin konvex gebogenen Mantellinien oder eierbecherförmig mit zur Rotationsachse (30) hin konkav gebogenen Mantellinien ausgebildet ist.

4. Endoprothese nach einem der Ansprüche 1 bis 3, **dadurch gekennzeichnet, dass** die Mantelfläche (25) wenigstens eine Ringkante (67) aufweist.

5. Endoprothese nach einem der Ansprüche 1 bis 4 in Verbindung mit Anspruch 2, **dadurch gekennzeichnet, dass** der Winkel des Kegelmantels (81) zur Rotationsachse (30) in einem Bereich liegt, innerhalb welchem eine Klemmwirkung zwischen der Mantelfläche (25) und der Anlenkfläche (43) erzielbar ist, vorzugsweise grösser 0° und kleiner 30°, besonders bevorzugt kleiner 15°.

6. Endoprothese nach einem der vorangehenden Ansprüche, **dadurch gekennzeichnet, dass** der Anlenkkörper (15) eine zentrale Bohrung (49) mit einem rotationssymmetrischen Grund (51) aufweist, das Rotationszentrum bzw. die Rotationsachse des rotationssymmetrischen Grunds (51) praktisch mit der Schwenkachse (45) zusammenfällt, und im Grund (51) des Anlenkkörpers (15) eine Öffnung (53) ausgebildet ist, und ein Befestigungsmittel (17) zum Verbinden von Anlenkkörper (15) und Grundteil (13) durch die Öffnung (53) hindurch vorhanden ist

7. Endoprothese nach Anspruch 6, **dadurch gekennzeichnet, dass** die Öffnung (53) ein Langloch ist, dessen Längserstreckung in Schwenkrichtung um die Schwenkachse (45) verläuft.

8. Endoprothese nach einem der vorangehenden Ansprüche, **dadurch gekennzeichnet, dass** das Befestigungsmittel (17) eine zur Rotationsachse (30) axiale Gewindeschraube (17) aufweist.

9. Endoprothese nach einem der Ansprüche 1 bis 8, **dadurch gekennzeichnet, dass** das Befestigungsmittel (17) ein Schraubdeckel, eine Anpressplatte oder eine Überwurfmutter aufweist.

10. Endoprothese nach einem der vorangehenden Ansprüche, **dadurch gekennzeichnet, dass** die Kreiskante (43) scharfkantig ausgebildet ist.

11. Endoprothese nach einem der Ansprüche 1 bis 9, **dadurch gekennzeichnet, dass** die Kreiskante (43) in einem radialen Schnitt gerundet ausgebildet ist.

12. Endoprothese nach einem der vorangehenden Ansprüche, **dadurch gekennzeichnet, dass** die Kreiskante (43) unterbrochen ist und insbesondere Spitzen oder Kerben aufweist.

13. Endoprothese nach einem der vorangehenden Ansprüche, **dadurch gekennzeichnet, dass** die Mantelfläche (25) in Umfangrichtung gerillt ist.

14. Endoprothese nach einem der vorangehenden Ansprüche, **dadurch gekennzeichnet, dass** die Mantelfläche (25) in Richtung der Mantellinien gerillt ist.

15. Endoprothese nach einem der vorangehenden Ansprüche, **dadurch gekennzeichnet, dass** die Mantelfläche (25) ein Rasterrelief aufweist.

16. Endoprothese nach Anspruch 12 und einem der Ansprüche 13 bis 15, **dadurch gekennzeichnet, dass** die Dimensionen der Spitzen oder Kerben der Kreiskanten (43) und die Dimensionen der Rillen oder des Rasterreliefs so aufeinander abgestimmt sind, dass sie sich miteinander verzahnen.

17. Endoprothese nach einem der vorangehenden Ansprüche, **dadurch gekennzeichnet, dass** die Mantelfläche (25) härter als die Kreiskante (43) ist.

18. Endoprothese nach einem der Ansprüche 1 bis 16, **dadurch gekennzeichnet, dass** die Mantelfläche (25) weicher als die Kreiskante (43) ist.

19. Endoprothese nach einem der vorangehenden Ansprüche, **dadurch gekennzeichnet, dass** der Öffnungsrand (27) der Anlenkmulde (21) eine den Öffnungsrand (27) umlaufende Verstärkungsrippe (57) aufweist.

## Claims

1. An endoprosthesis for replacing a joint, in particular a shoulder joint, having
- a base part (11) anchorable in a bone,
- a coupling cavity (21) arranged in the base part (11) and tapering towards the bottom (23) of the cavity, said coupling cavity (21) having a rotationally symmetrical lateral surface (25),
- a coupling member (15) in the coupling cavity (21), which coupling member (15) bears a first joint part (50) for articulation to a second natural or artificial joint part (52) and comprises a coupling surface (43), which cooperates with the lateral surface (25) of the coupling cavity (21) at contact points (55), which coupling surface (43) is of rotationally symmetrical construction at each contact point (55) relative to a single swivel axis (45) intersecting the axis of rotation (30),
- and having a fastening means (17) for fastening the coupling member (15) in the coupling cavity (21)
**characterised in that**
- the coupling surface (43) cooperates with the lateral surface (25) at at least three separate contact points (55),
- the coupling surface (43) comprises at least one circular edge (43) rotationally symmetrical about the swivel axis (45), which circular edge (43) cooperates with the lateral surface (25) at two of the at least three mutually spaced contact points (55),
- and one of these contact points (55) of the circular edge (43) is located on one side of a plane containing the swivel axis (45) and the axis of rotation (30) and the other contact point (55) of the circular edge (43) is located on the other side of this plane.

2. An endoprosthesis according to claim 1, **characterised in that** the radius (63) of the circular edge (43) is larger than the radius (65) of a vertex circle of an intersection curve (61) between a plane (89), lying perpendicular to the swivel axis (45), through the contact points (55) of the circular edge (43) and an envelope of a cone (81), formed by rotation about the axis of rotation (30) of a straight line (95) intersecting the axis of rotation (30) and a contact point (55), which straight line (95) belongs to a tangential plane (91) defined by a first tangent (85) and a second tangent (83) to the contact point (55), which first tangent (85) is tangent to the circular edge (43) in the plane (89) perpendicular to the swivel axis (45) and which second tangent (83) is tangent to the lateral surface (25) in a plane (87) perpendicular to the axis of rotation (30).

3. An endoprosthesis according to claim 1 or claim 2, **characterised in that** the lateral surface (25) is of conical construction with straight generating lines or trumpet-shaped with generating lines bent convexly towards the axis of rotation (30) or eggcup-shaped with generating lines bent concavely towards the axis of rotation (30) .

4. An endoprosthesis according to one of claims 1 to 3, **characterised in that** the lateral surface (25) has at least one annular edge (67).

5. An endoprosthesis according to one of claims 1 to 4 together with claim 2, **characterised in that** the angle of the cone envelope (81) to the axis of rotation (30) lies in an area within which a clamping action may be achieved between the lateral surface (25) and the coupling surface (43), preferably greater than 0° and smaller than 30°, particularly preferably smaller than 15°.

6. An endoprosthesis according to one of the preceding claims, **characterised in that** the coupling member (15) comprises a central bore (49) with a rotationally symmetrical bottom (51), the centre or axis of rotation of the rotationally symmetrical bottom (51) virtually coincides with the swivel axis (45), and an opening (53) is formed in the bottom (51) of the coupling member (15), and a fastening means (17) for connecting coupling member (15) and bottom part (13) through the opening (53) is present.

7. An endoprosthesis according to claim 6, **characterised in that** the opening (53) is an elongate hole, the longitudinal extent of which extends in the swivel direction about the swivel axis (45).

8. An endoprosthesis according to one of the preceding claims, **characterised in that** the fastening means (17) comprises a threaded screw (17) axial relative to the axis of rotation (30).

9. An endoprosthesis according to one of claims 1 to 8, **characterised in that** the fastening means (17) comprises a screw cap, a pressure plate or a union nut.

10. An endoprosthesis according to one of the preceding claims, **characterised in that** the circular edge (43) is of sharp-edged construction.

11. An endoprosthesis according to one of claims 1 to 9, **characterised in that** in a radial section the circular edge (43) is of rounded construction.

12. An endoprosthesis according to one of the preceding claims, **characterised in that** the circular edge (43) is interrupted and in particular comprises points or notches.

13. An endoprosthesis according to one of the preceding claims, **characterised in that** the lateral surface (25) is grooved in the circumferential direction.

14. An endoprosthesis according to one of the preceding claims, **characterised in that** the lateral surface (25) is grooved in the direction of the generating lines.

15. An endoprosthesis according to one of the preceding claims, **characterised in that** the lateral surface (25) has a grid relief.

16. An endoprosthesis according to claim 12 and one of claims 13 to 15, **characterised in that** the dimensions of the points or notches of the circular edges (43) and the dimensions of the grooves or of the grid relief are matched to one another in such a way that they mesh together.

17. An endoprosthesis according to one of the preceding claims, **characterised in that** the lateral surface (25) is harder than the circular edge (43).

18. An endoprosthesis according to one of claims 1 to 16, **characterised in that** the lateral surface (25) is softer than the circular edge (43).

19. An endoprosthesis according to one of the preceding claims, **characterised in that** the opening edge (27) of the coupling cavity (21) comprises a reinforcing rib (57) encircling the opening edge (27).

## Revendications

1. Endoprothèse pour la substitution d'une articulation, en particulier d'une articulation d'épaule, avec
- une pièce de base (11) pouvant être ancrée dans un os,
- une cuvette d'articulation (21), placée dans la pièce de base (11) en s'effilant vers le fond de la cuvette (23), avec une surface d'enveloppe à symétrie de rotation,
- un corps d'articulation (15) dans la cuvette d'articulation (21), lequel corps d'articulation (15) porte une première pièce d'articulation (50) pour l'articulation avec une seconde pièce d'articulation (52) naturelle ou artificielle et qui présente une surface d'articulation (43) qui coopère avec la surface d'enveloppe (25) de la cuvette d'articulation (21) à des endroits de contact (55), laquelle surface d'articulation (43) est configurée à chaque endroit de contact (55) à rotation symétrique par rapport à un seul axe de pivotement (45) qui coupe l'axe de rotation (30)
- et avec un moyen de fixation (17) pour fixer le corps d'articulation (15) dans la cuvette d'articulation (21),
**caractérisée en ce**
- **que** la surface d'articulation (43) coopère à au moins trois endroits de contact (55) séparés l'un de l'autre avec la surface d'enveloppe (25),
- **que** la surface d'articulation (43) présente au moins une arête circulaire (43) à rotation symétrique autour de l'axe de pivotement (45) qui coopère avec la surface d'enveloppe (25) à deux des trois endroits de contact (55) espacés l'un de l'autre qui existent au moins
- et **que** l'un de ces endroits de contact (55) de l'arête circulaire (43) est situé sur l'un des côtés d'un plan qui contient l'axe de pivotement (45) et l'axe de rotation (30) et que l'autre endroit de contact (55) de l'arête circulaire (43) est situé sur l'autre côté de ce plan.

2. Endoprothèse selon la revendication 1, **caractérisée en ce que** le rayon (63) de l'arête circulaire (43) est plus grand que le rayon (65) d'un cercle à point culminant d'une courbe d'intersection (61) entre un plan (89) situé perpendiculairement à l'axe de pivotement (45) à travers les endroits de contact (55) de l'arête circulaire (43) et une aire latérale de cône (81) formée par la rotation autour de l'axe de rotation (30) d'une droite (95) qui coupe l'axe de rotation (30) et un endroit de contact (55), laquelle droite (95) appartient à un plan tangentiel (91) défini par une première tangente (85) et une seconde tangente (83) à l'endroit de contact (55), laquelle première tangente (85) est tangente à l'arête circulaire (43) dans le plan (89) situé perpendiculairement à l'axe de pivotement (45) et laquelle seconde tangente (83) est tangente à la surface d'enveloppe (25) dans un plan (87) situé perpendiculairement à l'axe de rotation (30).

3. Endoprothèse selon la revendication 1 ou 2, **caractérisée en ce que** la surface d'enveloppe (25) est configurée conique avec des génératrices en ligne droite ou en forme de trompette avec des génératrices courbées convexes vers l'axe de rotation (30) ou en forme de coquetiers avec des génératrices courbées concaves vers l'axe de rotation (30).

4. Endoprothèse selon l'une des revendications 1 à 3, **caractérisée en ce que** la surface d'enveloppe (25) présente au moins une arête annulaire (67).

5. Endoprothèse selon l'une des revendications 1 à 4 en relation avec la revendication 2, **caractérisée en ce que** l'angle de l'aire latérale de cône (81) par rapport à l'axe de rotation (30) est situé dans une plage, à l'intérieur de laquelle un effet de serrage peut être obtenu entre la surface d'enveloppe (25) et la surface d'articulation (43), de préférence supérieure à 0° et inférieure à 30°, de manière particulièrement préférée inférieure à 15°.

6. Endoprothèse selon l'une des revendications précédentes, **caractérisée en ce que** le corps d'articulation (15) présente une forure centrale (49) avec un fond à symétrie de rotation (51), le centre de rotation ou l'axe de rotation du fond à symétrie de rotation (51) coïncide pratiquement avec l'axe de pivotement (45) et une ouverture (53) est configurée dans le fond (51) du corps d'articulation (15) et il existe un moyen de fixation (17) pour relier le corps d'articulation (15) et la pièce de base (13) à travers l'ouverture (53).

7. Endoprothèse selon la revendication 6, **caractérisée en ce que** l'ouverture (53) est un trou oblong dont l'extension en longueur est dans le sens de pivotement autour de l'axe de pivotement (45).

8. Endoprothèse selon l'une des revendications précédentes, **caractérisée en ce que** le moyen de fixation (17) présente une vis filetée (17) axiale par rapport à l'axe de rotation (30).

9. Endoprothèse selon l'une des revendications 1 à 8, **caractérisée en ce que** le moyen de fixation (17) présente un couvercle vissé, une plaque de pression ou un écrou-raccord.

10. Endoprothèse selon l'une des revendications précédentes, **caractérisée en ce que** l'arête circulaire (43) est configurée à arêtes vives.

11. Endoprothèse selon l'une des revendications 1 à 9, **caractérisée en ce que** l'arête circulaire (43) est configurée arrondie en une coupe radiale.

12. Endoprothèse selon l'une des revendications précédentes, **caractérisée en ce que** l'arête circulaire (43) est interrompue et présente en particulier des pointes ou des entailles.

13. Endoprothèse selon l'une des revendications précédentes, **caractérisée en ce que** la surface d'enveloppe (25) est rainurée dans le sens de la circonférence.

14. Endoprothèse selon l'une des revendications précédentes, **caractérisée en ce que** la surface d'enveloppe (25) est rainurée dans le sens des génératrices.

15. Endoprothèse selon l'une des revendications précédentes, **caractérisée en ce que** la surface d'enveloppe (25) présente un relief quadrillé.

16. Endoprothèse selon la revendication 12 et l'une des revendications 13 à 15, **caractérisée en ce que** les dimensions des pointes ou entailles des arêtes circulaires (43) et les dimensions des rainures ou du relief quadrillé sont adaptées les unes aux autres de telle manière qu'elles s'engrènent.

17. Endoprothèse selon l'une des revendications précédentes, **caractérisée en ce que** la surface d'enveloppe (25) est plus dure que l'arête circulaire (43).

18. Endoprothèse selon l'une des revendication 1 à 16, **caractérisée en ce que** la surface d'enveloppe (25) est plus souple que l'arête circulaire (43).

19. Endoprothèse selon l'une des revendications précédentes, **caractérisée en ce que** le bord de l'ouverture de la cuvette d'articulation (21) présente une nervure de renforcement (57) qui court sur le bord de l'ouverture (27).
